# EUROPÄISCHE PATENTSCHRIFT

(11) **EP 3 215 039 B1**
(45) Veröffentlichungstag und Bekanntmachung des Hinweises auf die Patenterteilung: **18.10.2017**
(21) Anmeldenummer: 16727680.7
(22) Anmeldetag: 03.06.2016
(51) Int. Cl.: A61B 17/70

(54) **OSTEOSYNTHESEVORRICHTUNG**
OSTEOSYNTHESIS DEVICE
DISPOSITIF D'OSTEOSYNTHESE

(30) Priorität: 29.07.2015 DE 102015214384
(43) Veröffentlichungstag der Anmeldung: 13.09.2017
(73) Patentinhaber: Silony Medical International AG, 8500 Frauenfeld (CH)
(72) Erfinder: HEUER, Frank, 70794 Filderstadt (DE)
(74) Vertreter: DREISS Patentanwälte PartG mbB
(86) Internationale Anmeldenummer: PCT/EP2016/062595
(87) Internationale Veröffentlichungsnummer: WO 2017/016717

(56) Entgegenhaltungen:
- EP-A2- 0 836 835
- EP-B1- 2 570 090
- WO-A1-00/72770
- FR-A1- 2 794 637

## Beschreibung

Die Erfindung betrifft eine Osteosynthesevorrichtung, insbesondere eine Pedikelschraube, mit den Merkmalen des Oberbegriffs des Anspruchs 1.

Derartige Osteosynthesevorrichtungen sind aus dem Stand der Technik vielfach bekannt. Osteosynthesevorrichtungen betreffen insbesondere das Gebiet der Wirbelsäulenchirurgie, um benachbarte Wirbelkörper auszurichten und in einer gewünschten Position relativ zueinander zu fixieren. Hierbei wird in benachbarte Wirbelkörper jeweils ein Knochenanker eingebracht, und zwar typischerweise als Knochenschraube eingeschraubt, und es werden dann diese benachbarten Knochenanker über ein Korrekturelement, typischerweise über einen Korrekturstab verbunden, der in dem Gabelkopf einer jeweiligen Osteosynthesevorrichtung in einer bestimmten und von einem Chirurgen erwünschten Position geklemmt wird. Auf diese Weise werden benachbarte Wirbelkörper ausgerichtet und in einer gewünschten Position relativ zueinander fixiert.

Eine derartige Osteosynthesevorrichtung ist beispielsweise aus der EP 2 301 458 A1 bekannt. Bei dieser aus dem Stand der Technik bekannten Knochenschraube wird der Knochenanker bzw. die Knochenschraube vom proximalen Endbereich des Gabelkopfs durch eine Aufnahmeöffnung hindurchgesteckt, so dass der Kopf verschwenkbar in einem verjüngten Bereich der Aufnahmeöffnung gelagert ist. Zur Fixierung der Knochenschraube in einer bestimmten, vom Chirurgen festgelegten Winkellage ist ein Druckstück vorgesehen, welches vom proximalen Ende in den Gabelkopf eingesetzt ist. Dieses Druckstück wird bei der Montage der Knochenschraube von dem Korrekturelement bzw. dem Korrekturstab durch Einschrauben einer Madenschraube in ein Gewinde des Gabelkopfs derart zum distalen Endbereich des Gabelkopfs hin beaufschlagt, dass es den Kopf des Knochenankers bzw. der Knochenschraube in seiner jeweiligen winkligen Lage relativ zum Gabelkopf fixiert. Zum Lösen dieser Winkelfixierung genügt ein Lösen der Madenschraube, so dass der axiale Druck, den das Korrekturelement bzw. des Korrekturstabs auf das Druckstück ausübt, derart vom Druckstück genommen werden kann, dass das Druckstück den Kopf des Knochenankers nicht mehr klemmt.

Nachteilig an derartigen Osteosynthesevorrichtungen ist jedoch, dass die Schaftdurchmesser der Knochenanker bzw. Knochenschrauben, die ein Außengewinde zum Einschrauben in einen Knochen oder Wirbelkörper aufweisen, in ihrer Größe begrenzt sind. So ist es nicht möglich, einen Knochenanker bzw. eine Knochenschraube mit einem Schaftdurchmesser, der größer ist als der Durchmesser des Kopfes, vom proximalen Ende des Gabelkopfs in die Aufnahmeöffnung einzusetzen. Für Anwendungen, die einen großen Schaftdurchmesser erfordern, lassen sich derartige Osteosynthesevorrichtungen daher nicht verwenden.

Aus dem Stand der Technik sind ferner Osteosynthesevorrichtungen bekannt, bei denen ein Kopf eines Knochenankers bzw. einer Knochenschraube von einem distalen Ende in den Gabelkopf der Osteosynthesevorrichtung eingesetzt sind. Eine derartige Osteosynthesevorrichtung ist aus der EP 2 570 090 B1 und der US 2009/0036934 A1 bekannt. Bei dieser Ostensynthesevorrichtung ist der Gabelkopf zweiteilig ausgebildet, wobei er einen ersten proximalen Teil mit den Schenkeln zur Aufnahme des Korrekturstabs aufweist und wobei er einen distalen Klemmteil zur Aufnahme des Kopfs der Knochenschraube aufweist, der an seiner Außenoberfläche konisch ausgebildet ist. Der distale Teil ist mittels zweier Stifte mit dem proximalen Teil drehbar verbunden. Der distale Klemmteil weist elastische Federelemente auf, die eine Aufnahmeöffnung für den Kopf der Knochenschraube begrenzen. Der Kopf der Knochenschraube ist vom distalen Ende in den distalen Klemmteil eingesetzt, wobei zur Sicherung des Kopfes ein radial außerhalb des distalen Klemmteils angeordneter Klemmring vorgesehen ist. Bei der Montage dieser Osteosynthesevorrichtung durch einen Chirurgen wird wiederum ein Korrekturstab zwischen die Schenkel des proximalen Teils eingelegt und von einer Madenschraube zum distalen Ende hin beaufschlagt. Dieser Korrekturstab beaufschlagt wiederum den Klemmring derart, dass er auf der konischen Außenoberfläche des Klemmteils so nach unten gedrückt wird, dass der Kopf der Knochenschraube in einer jeweiligen Winkellage fixiert wird.

Nachteilig an einer derartigen Osteosynthesevorrichtung ist jedoch, dass eine Korrektur der Winkeleinstellung durch einen Chirurgen nach dem erstmaligen Anziehen der Madenschraube nicht oder nur schwer möglich ist, da der Klemmring auf der konischen Außenoberfläche des Klemmteils bereits verklemmt ist. Ein Lösen der Madenschraube bewirkt somit nicht unmittelbar ein Lösen der Winkellage der Knochenschraube relativ zum Gabelkopf. Dies erweist sich insbesondere bei bereits in Knochen oder Wirbelkörper eingeschraubten Knochenschrauben als problematisch. Ferner ist die mehrteilige Ausbildung des Gabelkopfs kompliziert in der Montage und teuer in der Herstellung.

Die Erfindung stellt sich daher die Aufgabe, eine Osteosynthesevorrichtung bereitzustellen, mit der große Schaftdurchmesser der Knochenanker einfach und kostengünstig realisierbar sind, wobei eine einfache Korrektur der Winkellage des Knochenankers gewährleistet werden soll.

Diese Aufgabe wird durch eine Osteosynthesevorrichtung mit den Merkmalen des Anspruchs 1 gelöst. Diese Osteosynthesevorrichtung zeichnet sich dadurch aus, dass die Federabschnitte radial außen von einer konzentrisch zur Aufnahmeöffnung angeordneten Ringnut begrenzt werden, die eine Aufnahme für ein Sicherungselement bildet.

Vorzugsweise sind die Federabschnitte nach radial außen derart federnd ausgebildet, dass die Aufnahmeöffnung zum Einsetzten des Kopfs des Knochenankers kurzzeitig radial aufgeweitet wird. Wenn kein Sicherungselement in die Ringnut eingesetzt ist, können die Federabschnitte von einer Sperrlage in eine Einführlage bewegt werden. Besonders bevorzugt ist es dabei, wenn die Federabschnitte elastisch verformbar ausgebildet sind, so dass sie nach ihrer Verformung wieder in die Sperrlage zurückbewegt werden können. Vorteilhafterweise ist die Verbindung zwischen den Federabschnitten des Gabelkopfs und dem Kopf des Knochenankers als Biegeschnappverbindung und/oder Ringschnappverbindung ausgebildet.

Erfindungsgemäß ist ein Sicherungselement vorgesehen, das in der Ringnut verliersicher angeordnet ist.

Erfindungsgemäß ist ferner vorgesehen, dass die Ringnut zylindrisch ausgebildet ist. Eine zylindrische Ringnut erweist sich als besonders vorteilhaft, da durch axiales Einführen oder Einsetzen eines Sicherungselements in die Ringnut eine radiale Verlagerung der Federabschnitte weitgehend oder nahezu weitgehend verhindert werden kann, so dass durch Einsetzen des Sicherungselements lediglich ein Verlagern der Federabschnitte in die Einführlage verhindert werden kann, wobei ein Klemmen des Kopfs des Knochenankers durch Anordnen des Sicherungselements in der Ringnut vermieden werden kann.

Erfindungsgemäß ist darüber hinaus vorgesehen, dass das Sicherungselement als kreisringartige, vorzugsweise zylindrische Sperrhülse ausgebildet ist und in der Ringnut befestigt ist und verhindert, dass die Federabschnitte nach radial außen bewegt werden können. Hierzu weist die Sperrhülse vorzugweise einen Querschnitt auf, der gleich oder nahezu gleich dem Durchmesser der Ringnut ist. Die Sperrhülse korrespondiert dabei vorteilhafterweise derart mit der zylindrischen Ringnut, dass die Federabschnitte in der Sperrlage gesichert sind und bei in der Ringnut befestigter Sperrhülse nicht in die Freigabelage bewegt werden können, wobei gleichzeitig ein Herausfallen der Sperrhülse aus der Ringnut verhindert werden kann und wobei keine oder nahezu keine Klemmung des Kopfs des Knochenankers durch die Federabschnitte bzw. die Sperrhülse erfolgt. Besonders bevorzugt ist es daher, wenn ein Verklemmen des Kopfs des Knochenankers lediglich durch ein in den Gabelkopf vom proximalen Ende eingesetztes Druckstück erfolgt, das im Gabelkopf verpresst ist und bei der Montage eines Korrekturelements bzw. Korrekturstabs derart axial gegen den Kopf des Knochenankers beaufschlagt wird, dass dieser in seiner jeweiligen Winkelauslenkung fixiert wird.

Vorteilhafterweise erfolgt die Befestigung der Sperrhülse durch Einpressen, Verklemmen, Verkleben, Vernieten, Verschweißen oder Verschrauben. Es ist auch denkbar, dass die Ringnut und/oder die Sperrhülse wenigstens eine konische Fläche aufweisen, die derart ausgebildet ist, dass eine verliersichere Befestigung der Sperrhülse in der Ringnut erleichtert ist.

Eine weitere vorteilhafte Ausgestaltung der Osteosynthesevorrichtung sieht vor, dass die Aufnahmeöffnung nach radial außen von einem Kreisringabschnitt begrenzt wird, der die Federabschnitte bildet. Vorteilhafterweise ist der Kreisringabschnitt dann zwischen der Aufnahmeöffnung und der Ringnut angeordnet.

Besonders vorteilhaft ist es dabei, wenn der Kreisringabschnitt über seinen Umfang angeordnete Ausnehmungen aufweist, die den Kreisringabschnitt in die Federabschnitte unterteilen. Vorteilhafterweise sind die Federabschnitte am proximalen Ende mit dem Gabelkopf einstückig verbunden und weisen am distalen Ende ein freies Ende auf. Vorzugsweise sind mehrere, weiter vorzugsweise 4 bis 8, weiter vorzugsweise 6 Ausnehmungen vorgesehen.

In einer bevorzugten Weiterbildung der Osteosynthesevorrichtung ist vorgesehen, dass die Ausnehmungen parallel zu einer Mittenachse der Aufnahmeöffnung verlaufen. Dabei ist denkbar, dass die Ausnehmungen zylindrisch ausgebildet sind.

In einer weiteren besonders vorteilhaften Weiterbildung der Osteosynthesevorrichtung ist vorgesehen, dass die Aufnahmeöffnung einen Öffnungsdurchmesser aufweist und dass der Schaft des Knochenankers einen Außendurchmesser aufweist, wobei der Öffnungsdurchmesser der Aufnahmeöffnung kleiner ist als der größte Außendurchmesser des Schafts. Mit einer derartigen Osteosynthesevorrichtung, kann folglich ein Knochenanker mit einem Schaft mit großem Außendurchmesser realisiert werden, wobei der Knochenanker beispielsweise auch in spröden Knochen oder Wirbelkörpern sicher befestigt werden kann.

Als besonders vorteilhaft hat es sich ferner erwiesen, wenn der Kopf des Knochenankers kugelig ausgebildet ist und wenn die Federabschnitte radial zur Aufnahmeöffnung hin ragende, Haltevorsprünge aufweisen. Vorteilhafterweise sind die Haltevorsprünge derart ausgebildet, dass sie ein Herausfallen des Kopfs aus dem Knochenanker in axialer Richtung verhindern.

Besonders vorteilhaft ist es dabei, wenn die Haltevorsprünge einen Gleitabschnitt aufweisen, der derart ausgelegt ist, dass der Kopf des Knochenankers verschwenkbar ist. Vorteilhafterweise weisen die Haltevorsprünge eine dem distalen Ende zugewandte, konkav ballige Fläche auf, die derart ausgebildet ist, dass der Kopf einerseits verschwenkbar im Gabelkopf gelagert ist und andererseits ein Herausfallen des Kopfs aus dem Gabelkopf verhindert ist.

Besonders bevorzugt ist es ferner, wenn die Federabschnitte am distalen Endbereich eine der Aufnahmeöffnung radial zugewandte Einführschräge aufweisen. Mittels einer derartigen Einführschräge kann ein Eindrücken oder Einpressen des Kopfs des Knochenankers bei der Montage der Osteosynthesevorrichtung erleichtert werden.

Weiterhin ist es vorteilhaft, wenn die Federabschnitte in axialer Richtung unterschiedlich lang ausgebildet sind. Vorteilhafterweise sind die Federabschnitte unsymmetrisch derart ausgebildet, dass in einer ersten Auslenkungsrichtung eine größere Auslenkung realisiert werden kann als in einer von der ersten Auslenkungsrichtung verschiedenen zweiten Auslenkungsrichtung.

Weitere Einzelheiten und vorteilhafte Weiterbildungen sind der nachfolgenden Beschreibung zu entnehmen, anhand derer die in den Figuren dargestellte Ausführungsform näher beschrieben und erläutert ist.

Es zeigen:
Figur 1 eine Schrägansicht von unten auf eine erfindungsgemäße Osteosynthesevorrichtung in einer Explosionsdarstellung;
Figur 2 eine Seitenansicht der Osteosynthesevorrichtung gemäß Figur 1 in Explosionsdarstellung;
Figur 3 die Seitenansicht der Osteosynthesevorrichtung gemäß Figur 2 in nicht explodierter Darstellung;
Figur 4 einen Schnitt durch die Osteosynthesevorrichtung entlang der Linie A-A gemäß Figur 3;
Figur 5 eine Schrägansicht eines Gabelkopfs der Osteosynthesevorrichtung gemäß der Figuren 1 bis 4;
Figur 6 eine Seitenansicht des Gabelkopfs gemäß Figur 5; und
Figur 7 einen Schnitt durch den Gabelkopf entlang der Linie B-B gemäß Figur 6.

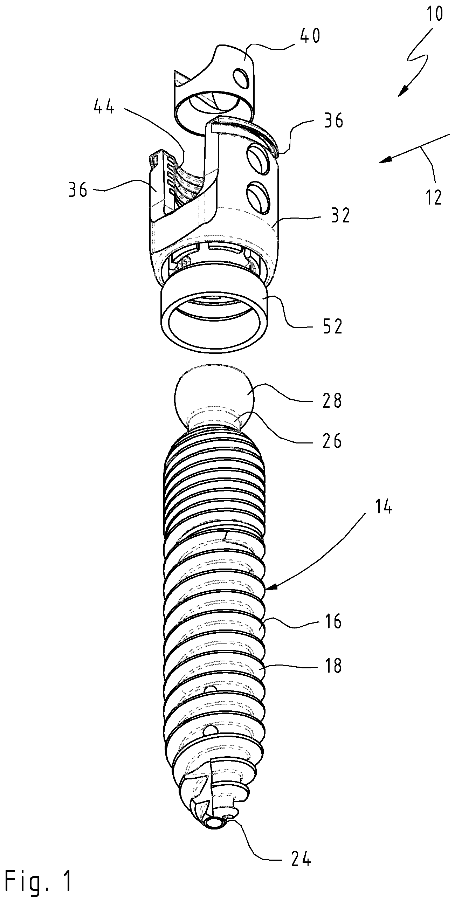

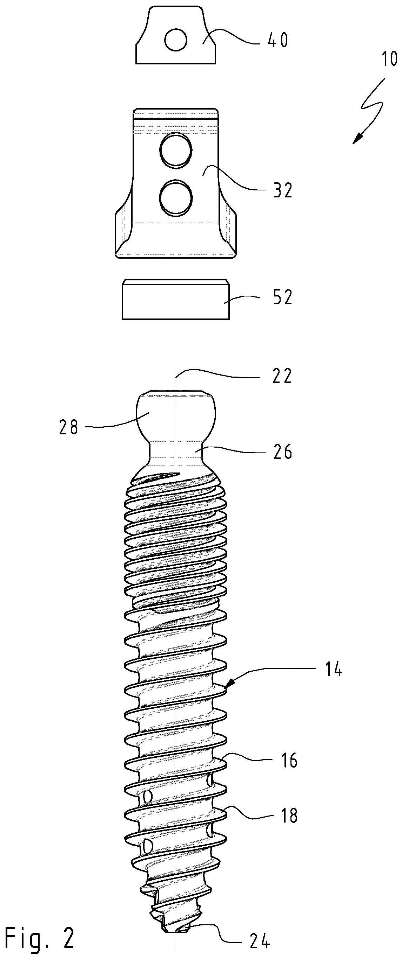

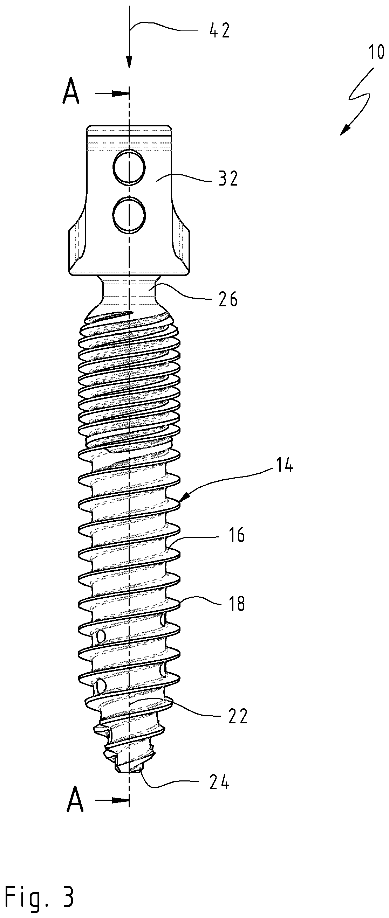

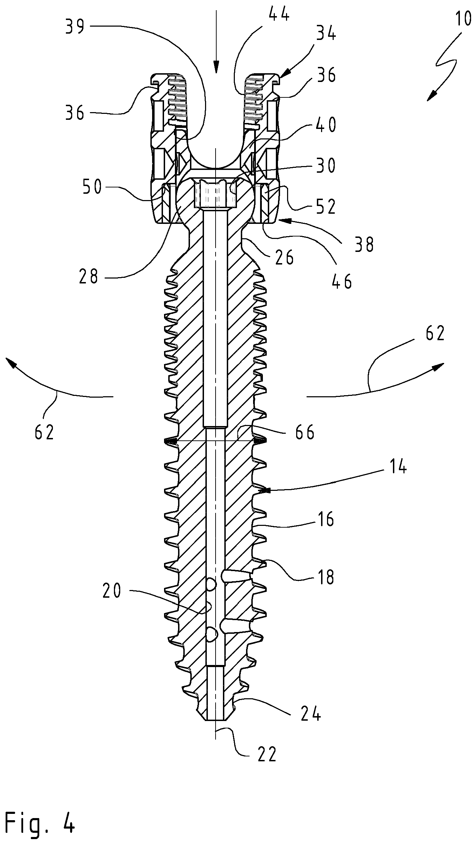

Die Figuren 1 bis 4 zeigen eine insgesamt mit dem Bezugszeichen 10 bezeichnete, als Pedikelschraube, insbesondere als Polyaxialschraube ausgebildete erfindungsgemäße Osteosynthesevorrichtung 10. Sich in den Figuren entsprechende Bauteile und Elemente sind dabei mit sich entsprechenden Bezugszeichnen gekennzeichnet. Figur 1 zeigt die Osteosynthesevorrichtung 10 in einer Schrägansicht von unten. Figur 2 zeigt die Osteosynthesevorrichtung 10 in einer Seitenansicht bei Blick in Richtung des Pfeils 12 in Figur 1 in einer Explosionsdarstellung. Figur 3 zeigt die Osteosynthesevorrichtung 10 in der Seitenansicht gemäß Figur 2 in einer nicht explodierten Darstellung und Figur 4 zeigt einen Schnitt durch die Osteosynthesevorrichtung 10 entlang der Linie A-A gemäß Figur 3.

Die Osteosynthesevorrichtung 10 umfasst einen als Knochenschraube ausgebildeten Knochenanker 14, der einen Schaft 16 mit einem Außengewinde 18 aufweist. Der Knochenanker 14 bzw. dessen Schaft 16 ist kanüliert ausgebildet, d.h. er weist eine durchgehende Längsbohrung 20 auf, die konzentrisch zu einer Mittellängsachse 22 des Knochenankers 14 bzw. des Schafts 16 angeordnet ist. Der Knochenanker 14 kann ferner in den Figuren nicht dargestellte Querbohrungen aufweisen, die mit der Längsbohrung 20 verbunden sind. Mittels der Längsbohrung 20 und der Querbohrungen kann Knochenzement zwischen dem Schaft 16 und einem Knochen eines Patienten ausgebracht werden. Der Schaft 16 weist an einem Ende eine Bohrspitze 24 auf, mittels derer der Knochenanker 14 in einen Knochen eingeschraubt werden kann. An seinem der Bohrspitze 24 abgewandten Ende weist der Knochenanker 14 einen als Einschnürung ausgebildeten Hals 26 auf, an den sich in Richtung der Mittellängsachse 22 ein Kopf 28 des Knochenankers 14 anschließt. Im Kopf 28 weist der Knochenanker 14 ferner einen Drehmomentübertragungsabschnitt 30 in Form eines Sechskantprofils oder Torxprofils auf, der konzentrisch zur Mittellängsachse 22 angeordnet ist und mittels dessen der Knochenanker 14 rotierend angetrieben werden kann, so dass er in einen Knochen eines Patienten eingeschraubt werden kann.

Die Osteosynthesevorrichtung 10 umfasst ferner einen einstückig ausgebildeten Gabelkopf 32. Der Gabelkopf 32 dient zur Aufnahme des Knochenankers 14, wobei der Gabelkopf 32 in einer Seitenansicht, die im Schnitt gemäß Figur 4 erkennbar ist, U-förmig ausgebildet ist und in einem proximalen Endbereich 34 zwei Schenkel 36 aufweist. Der Kopf 28 des Knochenankers 14 ist an einem den Schenkeln 36 abgewandten distalen Endbereich 38 des Gabelkopfs 32 nach Art eines Kalottenlagers verschwenkbar gelagert und kann in verschiedenen, von einem Chirurgen einzustellenden Positionen relativ zum Gabelkopf 32 festgelegt werden.

Hierfür weist die Osteosynthesevorrichtung 10 im Inneren 39 des Gabelkopfs 32 beweglich verpresstes Druckstück 40 auf, das axial in einem definierten Bereich beweglich ist. Zur Festlegung einer Verschwenkung des Knochenankers 14 wird vom proximalen Endbereich 34 des Gabelkopfs 32 ausgehend, mittels nicht dargestellter Klemmelemente, wie bspw. einer Madenschraube und eines im Gabelkopf 32 zwischen den Schenkeln 36 angeordneten, ebenfalls nicht dargestellten Korrekturstabs eine Klemmkraft auf das Druckstück 40 in Richtung eines Pfeils 42 ausgeübt, so dass das Druckstück 40 wiederum auf den Kopf 28 des Knochenankers 14 drückt.

Zur Aufnahme eines als bspw. Madenschraube ausgebildeten Klemmelements weist der Gabelkopf 32 im proximalen Endbereich 34 im Inneren 39 ein Innengewinde 44 auf. Vor dem Anziehen des Klemmelements wird zwischen dem Kopf 28 des Knochenankers 14 und dem Klemmelement ein an sich bekannter Korrekturstab eingelegt, wobei durch Festziehen des Klemmelements die gesamte Anordnung fixierbar ist, was typischerweise erst geschieht, wenn der Knochenanker 14 in seiner bestimmungsgemäßen Position in den Knochen eines Patienten eingebracht ist. Nach Einbringen des Knochenankers 14 werden der Gabelkopf 32 und der in das Innere 39 des Gabelkopfs 32 eingelegte Korrekturstab relativ zum Knochenanker 14 ausgerichtet und in der vom Chirurgen intendierten Position durch Festziehen des Klemmelements fixiert.

Der Gabelkopf 32 weist am distalen Endbereich 38 eine Aufnahmeöffnung 46 für den Kopf 28 des Knochenankers 14 auf, wobei der Kopf 28 des Knochenankers 14 vom distalen Ende des Gabelkopfs 32 in die Aufnahmeöffnung 46 eingesetzt ist.

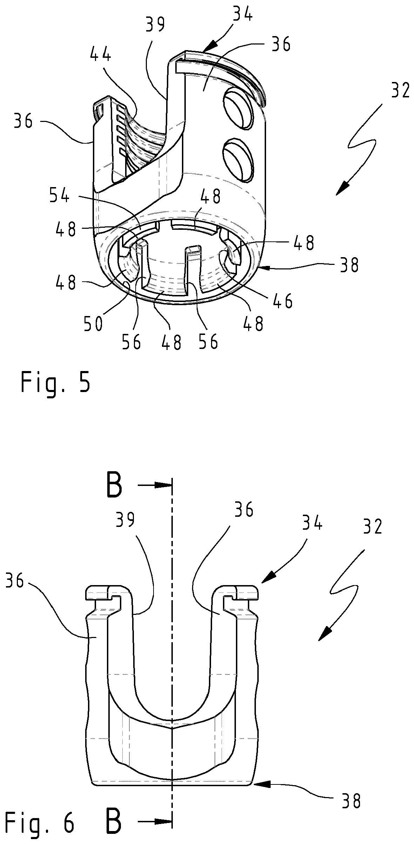

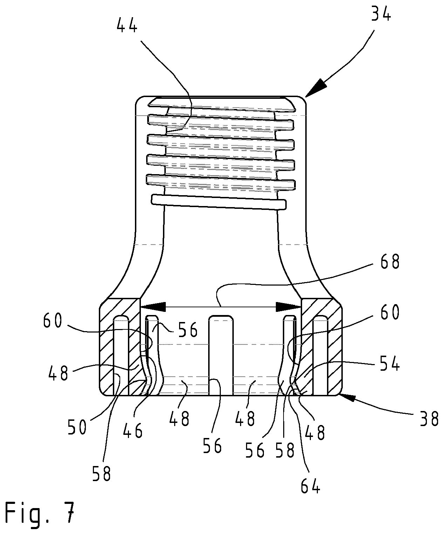

Der Gabelkopf 32 ist in den Figuren 5 bis 7 in Alleindarstellung gezeigt, wobei die Funktionsweise der erfindungsgemäßen Osteosynthesevorrichtung 10 anhand der Figuren 4 bis 7 näher beschrieben und erläutert wird. Der Gabelkopf 32 weist an seinem distalen Endbereich die Aufnahmeöffnung 46 für den Kopf 28 des Knochenankers 14 auf. Die Aufnahmeöffnung 46 wird in radialer Richtung von elastischen Federabschnitten 48 begrenzt, die wiederum radial außen von einer konzentrisch zur Aufnahmeöffnung 46 angeordneten, zylindrischen Ringnut 50 begrenzt werden.

In die Ringnut 50 ist ein als kreisringartige zylindrische Sperrhülse ausgebildetes Sicherungselement 52 eingepresst, das in den Figuren 1, 2 und 4 deutlich zu erkennen ist. Das Sicherungselement 52 korrespondiert derart mit der Ringnut 50, dass es zwar in der Ringnut 50 verliersicher angeordnet ist, jedoch keine oder nahezu keine radiale Klemmkraft auf die Federabschnitte 48 ausübt. In einer weiteren, in den Figuren nicht gezeigten Ausführungsform kann das Sicherungselement 52 auch als ovale Sperrhülse ausgebildet sein, wobei die Nut ebenfalls oval ausgebildet sein kann, so dass eine Rotationshemmung der Sperrhülse in der ovalen Nut erreicht werden kann.

Die Aufnahmeöffnung 46 wird radial außen von einem Kreisringabschnitt 54 begrenzt, der die Federabschnitte 48 bildet. Hierzu weist der Kreisringabschnitt 54 über seinen Umfang angeordnete Ausnehmungen 56 auf, die parallel zu einer Mittenachse der Aufnahmeöffnung 46 verlaufen und den Kreisringabschnitt 54 in die Federabschnitte 48 unterteilen.

Ferner ist der Kopf 28 des Knochenankers 14 kugelig ausgebildet, wobei die Federabschnitte 48 radial zur Aufnahmeöffnung 46 hin ragende Haltevorsprünge 58 aufweisen, die ein Herausfallen des Kopfs 28 des Knochenankers 14 aus der Aufnahmeöffnung 46 verhindern. Die Haltevorsprünge 58 weisen einen Gleitabschnitt 60 auf, der derart ausgelegt ist, dass der Kopf 28 des Knochenankers 14 in Richtung des Doppelpfeils 62 verschwenkbar ist. Der Gleitabschnitt 14 ist konkav ballig ausgebildet, so dass der kugelige Kopf 28 des Knochenankers 14 auf ihm gleiten kann. Die Federabschnitte 48 weisen am distalen Endbereich 38 eine der Aufnahmeöffnung 46 radial zugewandte Einführschräge 64 auf.

Der Schaft 16 des Knochenankers 14 weist am Außengewinde 16 einen Außendurchmesser 66 auf, wobei die Aufnahmeöffnung 46 einen Öffnungsdurchmesser 68 aufweist. Der Außendurchmesser 66 des Schafts 16 ist an seiner größten Abmessung größer als der Innendurchmesser der Aufnahmeöffnung 46, so dass der Knochenanker 14 nicht von oben, d.h. vom proximalen Endbereich 34 her durch die Aufnahmeöffnung 46 hindurchgesteckt werden kann.

Die Montage der Osteosynthesevorrichtung 10 erfolgt folgendermaßen:
Der Knochenanker 14 wird vom distalen Endbereich 34 des Gabelkopfs 32 derart in die Aufnahmeöffnung 46 des Gabelkopfs 32 eingedrückt oder eingepresst, dass die elastischen Federabschnitte 48 nach radial außen in eine Einführlage bewegt werden und in die Ringnut 50 ausweichen. Zu diesem Zeitpunkt ist das Sicherungselement 52 noch nicht in der Ringnut 50 montiert. Nach dem Eindrücken oder Einpressen des Kopfs 28 des Knochenankers 14 werden die Federabschnitte elastisch in die in Figur 4 dargestellte Sperrlage zurückbewegt, wobei die Haltevorsprünge 58 den Kopf 28 des Knochenankers 14 axial derart hinterschneiden, dass ein Herausfallen des Knochenankers 14 aus der Aufnahmeöffnung 46 verhindert ist.

Nach dem Eindrücken oder Einpressen des Kopfs 28 des Knochenankers 14 in die Aufnahmeöffnung 46 werden die Federabschnitte 48 in der Sperrlage durch Anordnen des Sicherungselements 52 in der Ringnut 50 gesichert. Dabei verhindert das Sicherungselement 52, das beispielsweise durch Einpressen verliersicher in der Ringnut 50 angeordnet wird, lediglich, dass die Federabschnitte 48 nach radial außen in die Freigabelage bewegt werden können. Ein Ausüben von radialer Klemmkraft auf den Kopf 28 des Knochenankers 14 durch die Federabschnitte 48 ist erfindungsgemäß nicht erwünscht.

Zur Festlegung einer axialen Verschwenkung des Knochenankers 14 relativ zum Gabelkopf 32 wird, wie eingangs beschrieben, vom proximalen Endbereich 34 eine Klemmkraft durch nicht dargestellte Klemmelemente bzw. einen nicht dargestellten Korrekturstab auf das Druckstück 40 ausgeübt, welches wiederum den Kopf 28 des Knochenankers in seiner jeweiligen, von einem Chirurgen festgelegten Lage sichert.

Zur Realisierung einer unsymmetrischen Auslenkung des Knochenankers 14 kann ferner vorgesehen sein, die Federabschnitte 48 beispielsweise unsymmetrisch derart auszubilden, dass in einer Richtung eine größere Auslenkung realisiert werden kann als in eine gegenüberliegende Richtung.

## Patentansprüche

1. Osteosynthesevorrichtung (10), insbesondere Pedikelschraube, mit einem einen Schaft (16) und einen Kopf (28) aufweisenden Knochenanker (14), insbesondere einer Knochenschraube, und mit einem in einer Seitenansicht U-förmigen Gabelkopf (32), der an einem proximalen Endbereich (34) zwei Schenkel (36) aufweist und einstückig ausgebildet ist, wobei der Kopf (28) des Knochenankers (14) an einem den Schenkeln (36) abgewandten distalen Endbereich (38) des Gabelkopfs (32) verschwenkbar gelagert ist, wobei der Gabelkopf (32) am distalen Endbereich (38) eine von Federabschnitten (48) in radialer Richtung begrenzte Aufnahmeöffnung (46) für den Kopf (28) des Knochenankers (14) aufweist, wobei die Federabschnitte (48) radial innen von der Aufnahmeöffnung (46) begrenzt werden, wobei der Kopf (28) des Knochenankers (14) vom distalen Ende des Gabelkopfs (32) her in die Aufnahmeöffnung (46) eingesetzt ist, **dadurch gekennzeichnet, dass** die Federabschnitte (48) radial außen von einer konzentrisch zur Aufnahmeöffnung (46) angeordneten Ringnut (50) begrenzt werden, die eine Aufnahme für ein Sicherungselement (52) bildet, wobei die Ringnut (50) zylindrisch ausgebildet ist, wobei ein Sicherungselement (52) vorgesehen ist, das in der Ringnut (50) verliersicher angeordnet ist und wobei das Sicherungselement (52) als kreisringartige vorzugsweise zylindrische Sperrhülse ausgebildet ist und in der Ringnut (50) befestigt ist und verhindert, dass die Federabschnitte (48) nach radial außen bewegt werden können.

2. Osteosynthesevorrichtung (10) Anspruch 1, **dadurch gekennzeichnet, dass** die Aufnahmeöffnung (46) nach radial außen von einem Kreisringabschnitt (54) begrenzt wird, der die Federabschnitte (48) bildet.

3. Osteosynthesevorrichtung (10) nach Anspruch 2, **dadurch gekennzeichnet, dass** der Kreisringabschnitt (54) über seinen Umfang angeordnete Ausnehmungen (56) aufweist, die den Kreisringabschnitt (54) in die Federabschnitte (48) unterteilen.

4. Osteosynthesevorrichtung (10) nach Anspruch 3, **dadurch gekennzeichnet, dass** die Ausnehmungen (56) parallel zu einer Mittenachse der Aufnahmeöffnung (46) verlaufen.

5. Osteosynthesevorrichtung (10) nach wenigstens einem der vorhergehenden Ansprüche, **dadurch gekennzeichnet, dass** die Aufnahmeöffnung (46) einen Öffnungsdurchmesser (68) aufweist und dass der Schaft (16) des Knochenankers (14) einen Außendurchmesser (66) aufweist, wobei der Öffnungsdurchmesser (68) der Aufnahmeöffnung (46) kleiner ist als der größte Außendurchmesser (66) des Schafts (16).

6. Osteosynthesevorrichtung (10) nach wenigstens einem der vorhergehenden Ansprüche, **dadurch gekennzeichnet, dass** der Kopf (28) des Knochenankers (14) kugelig ausgebildet ist und dass die Federabschnitte (48) radial zur Aufnahmeöffnung (46) hin ragende, Haltevorsprünge (58) aufweisen.

7. Osteosynthesevorrichtung (10) nach Anspruch 6, **dadurch gekennzeichnet, dass** die Haltevorsprünge (58) einen Gleitabschnitt (60) aufweisen, der derart ausgelegt ist, dass der Kopf (28) des Knochenankers (14) verschwenkbar ist.

8. Osteosynthesevorrichtung (10) nach wenigstens einem der vorhergehenden Ansprüche, **dadurch gekennzeichnet, dass** die Federabschnitte (48) am distalen Endbereich (38) eine der Aufnahmeöffnung (46) radial zugewandte Einführschräge (64) aufweisen.

9. Osteosynthesevorrichtung (10) nach wenigstens einem der vorhergehenden Ansprüche, **dadurch gekennzeichnet, dass** die Federabschnitte (48) in axialer Richtung unterschiedlich lang ausgebildet sind.

## Claims

1. Osteosynthesis device (10), in particular a pedicle screw, with a bone anchor (18) having a shaft (16) and a head (28), in particular a bone screw, and with a fork head (32) which is U-shaped in side view and has two legs (36) at a proximal end region (34) and is formed from one piece, wherein the head (28) of the bone anchor (14) is mounted pivotably at a distal end region (38) of the fork head (32) facing away from the legs (36), wherein the fork head (32) at the distal end region (38) has an opening (46) delimited in the radial direction by spring portions (48) for receiving the head (28) of the bone anchor (14), wherein the spring portions (48) are delimited radially inwardly by the receiving opening (46), wherein the head (28) of the bone anchor (14) is inserted in the receiving opening (46) from the distal end of the fork head (32), **characterised in that** the spring portions (48) are delimited radially outwardly by a ring groove (50) arranged concentrically to the receiving opening (46) and forming a receiver for a securing element (52), wherein the ring groove (50) is formed so as to be cylindrical, wherein a securing element (52) is provided which is arranged captively in the ring groove (50) and wherein the securing element (52) is configured as an annular, preferably cylindrical blocking sleeve and is secured in the ring groove (50) and prevents the spring portions (48) from being able to move radially outwardly.

2. Osteosynthesis device (10) according to claim 1, **characterised in that** the receiving opening (46) is delimited radially outwardly by a circular ring portion (54) forming the spring portions (48).

3. Osteosynthesis device (10) according to claim 2, **characterised in that** the ring portion (54) has recesses (56) arranged over its periphery which divide the ring portion (54) into the spring portions (48).

4. Osteosynthesis device (10) according to claim 3, **characterised in that** the recesses (56) run parallel to a centre axis of the receiving opening (46).

5. Osteosynthesis device (10) according to at least one of the preceding claims, **characterised in that** the receiving opening (46) has an opening diameter (68), and that the shaft (16) of the bone anchor (14) has an outer diameter (66), wherein the opening diameter (68) of the receiving opening (46) is smaller than the largest outer diameter (66) of the shaft (16).

6. Osteosynthesis device (10) according to at least one of the preceding claims, **characterised in that** the head (28) of the bone anchor (14) is formed as a ball, and the spring portions (48) have retaining protrusions (58) protruding radially towards the receiving opening (46).

7. Osteosynthesis device (10) according to claim 6, **characterised in that** the retaining protrusions (58) have a slide portion (16) which is configured such that the head (28) of the bone anchor (14) is pivotable.

8. Osteosynthesis device (10) according to at least one of the preceding claims, **characterised in that** at the distal end region (38), the spring portions (48) have an insert chamfer (64) facing radially towards the receiving opening (46).

9. Osteosynthesis device (10) according to at least one of the preceding claims, **characterised in that** the spring portions (48) are formed with different lengths in the axial direction.

## Revendications

1. Dispositif d'ostéosynthèse (10), en particulier vis pédiculaire, comprenant un ancrage osseux (14) présentant une tige (16) et une tête (28), en particulier une vis à os, et comprenant une tête fourchue en forme de U (32) selon une vue latérale, qui présente deux branches (36) au niveau d'une zone d'extrémité proximale (34) et est conçue d'un seul bloc, la tête (28) de l'ancrage osseux (14) étant montée pivotante sur une zone d'extrémité distale (38), opposée aux branches (36), de la tête fourchue (32), la tête fourchue (32) présentant au niveau de la zone d'extrémité distale (38) une ouverture de réception (46), délimitée par des parties élastiques (48) dans la direction radiale, pour la tête (28) de l'ancrage osseux (14), les parties élastiques (48) étant délimitées radialement vers l'intérieur par l'ouverture de réception (46), la tête (28) de l'ancrage osseux (14) étant insérée par l'extrémité distale de la tête fourchue (32) dans l'ouverture de réception (46), **caractérisé en ce que** les parties élastiques (48) sont délimitées radialement à l'extérieur par une rainure annulaire (50) qui est agencée de manière concentrique par rapport à l'ouverture de réception (46) et qui forme un logement pour un élément de blocage (52), la rainure annulaire (50) étant cylindrique, un élément de blocage (52) étant prévu, lequel est agencé de manière imperdable dans la rainure annulaire (50) et l'élément de blocage (52) étant réalisé sous la forme d'un manchon d'arrêt du type anneau circulaire, de préférence cylindrique, et étant fixé dans la rainure annulaire (50) et empêchant que les parties élastiques (48) puissent être déplacées radialement vers l'extérieur.

2. Dispositif d'ostéosynthèse (10) selon la revendication 1, **caractérisé en ce que** l'ouverture de réception (46) est délimitée radialement vers l'extérieur par une partie formant anneau circulaire (54), laquelle forme les parties élastiques (48).

3. Dispositif d'ostéosynthèse (10) selon la revendication 2, **caractérisé en ce que** la partie formant anneau circulaire (54) présente des évidements (56) agencés sur son pourtour, lesquels subdivisent la partie formant anneau circulaire (54) en parties élastiques (48).

4. Dispositif d'ostéosynthèse (10) selon la revendication 3, **caractérisé en ce que** les évidements (56) s'étendent parallèlement à un axe central de l'ouverture de réception (46).

5. Dispositif d'ostéosynthèse (10) selon au moins l'une des revendications précédentes, **caractérisé en ce que** l'ouverture de réception (46) présente un diamètre (68) et **en ce que** la tige (16) de l'ancrage osseux (14) présente un diamètre extérieur (66), le diamètre (68) de l'ouverture de réception (46) étant inférieur au plus grand diamètre extérieur (66) de la tige (16).

6. Dispositif d'ostéosynthèse (10) selon au moins l'une des revendications précédentes, **caractérisé en ce que** la tête (28) de l'ancrage osseux (14) est sphérique et **en ce que** les parties élastiques (48) présentent des parties saillantes de retenue (58) faisant radialement saillie de l'ouverture de réception (46).

7. Dispositif d'ostéosynthèse (10) selon la revendication 6, **caractérisé en ce que** les parties saillantes de retenue (58) présentent une partie coulissante (60), laquelle est dimensionnée de telle sorte que la tête (28) de l'ancrage osseux (14) puisse pivoter.

8. Dispositif d'ostéosynthèse (10) selon au moins l'une des revendications précédentes, **caractérisé en ce que** les parties élastiques (48) présentent au niveau de la zone d'extrémité distale (38) un biseau d'insertion (64) orienté radialement vers l'ouverture de réception (46).

9. Dispositif d'ostéosynthèse (10) selon au moins l'une des revendications précédentes, **caractérisé en ce que** les parties élastiques (48) sont plus ou moins longues dans la direction axiale.
